(19) Europäisches Patentamt
European Patent Office
Office européen des brevets

(11) **EP 3 654 340 A1**

(12) **EUROPEAN PATENT APPLICATION**

(43) Date of publication:
**20.05.2020 Bulletin 2020/21**

(51) Int Cl.:
***G16H 20/10*** *(2018.01)*

(21) Application number: **18206265.3**

(22) Date of filing: **14.11.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
- **SREENIVASAN, Rithesh
5656 AE Eindhoven (NL)**
- **RAMASAMY, Rose
5656 AE Eindhoven (NL)**
- **BHAT, Shiva Moorthy Pookala Vittal
5656 AE Eindhoven (NL)**

(74) Representative: **de Haan, Poul Erik
Philips International B.V.
Philips Intellectual Property & Standards
High Tech Campus 5
5656 AE Eindhoven (NL)**

(54) **SYSTEM AND METHODS FOR AN ARTIFICIAL INTELLIGENCE (AI) BASED APPROACH FOR PREDICTIVE MEDICATION ADHERENCE INDEX (MAI)**

(57) A method for training an adherence model, the method including: extracting data for a group of individuals (510), wherein the extracted data includes demographic data (205) and clinical data (210); training a linear regression model (520) using a set of hyperparameter pairs (LI, Alpha) (515), wherein the linear regression model produces an adherence index based upon the extracted data, further including: for each hyperparameter pair (LI, Alpha) in the set of hyperparameter pairs, training the linear regression model using a training data set to produce a linear regression model for each hyperparameter pair (LI, Alpha) and calculating a performance metric R2 for the resulting model based upon a validation data set (525), wherein the training data set is a subset of the extracted data and the validation data set is a subset of the extracted data that is different from the training data set; and identifying the linear regression model with the largest performance metric R2 (530).

FIG. 5

EP 3 654 340 A1

**Description**

FIELD OF THE INVENTION

Various exemplary embodiments disclosed herein relate generally to systems and methods for an artificial intelligence (AI) based approach for predictive Medication Adherence Index (MAI).

BACKGROUND OF THE INVENTION

At any point of time, it is estimated that up to 50% of patients are not adherent to their prescribed medication. This can adversely affect the patient's health contributing to high healthcare costs. Identifying patients who are at high risk of non-adherence to medication is a challenge as it depends on various factors like clinical history, patient demographics, and patient socio-economic factors.

SUMMARY OF THE INVENTION

A summary of various exemplary embodiments is presented below. Some simplifications and omissions may be made in the following summary, which is intended to highlight and introduce some aspects of the various exemplary embodiments, but not to limit the scope of the invention. Detailed descriptions of an exemplary embodiment adequate to allow those of ordinary skill in the art to make and use the inventive concepts will follow in later sections.
Various embodiments relate to a method for training an adherence model, the method including: extracting data for a group of individuals, wherein the extracted data includes demographic data and clinical data; training a linear regression model using a set of hyperparameter pairs (L1, Alpha), wherein the linear regression model produces an adherence index based upon the extracted data, further including: for each hyperparameter pair (L1, Alpha) in the set of hyperparameter pairs, training the linear regression model using a training data set to produce a linear regression model for each hyperparameter pair (L1, Alpha) and calculating a performance metric R2 for the resulting model based upon a validation data set, wherein the training data set is a subset of the extracted data and the validation data set is a subset of the extracted data that is different from the training data set; and identifying the linear regression model with the largest performance metric R2.
Various embodiments are described, wherein the performance metric R2 is a measure the proportion of the variance in the adherence index that is predictable from the extracted data.
Various embodiments are described, wherein training the linear regression model uses a grid search wherein the set of hyperparameter pairs are generated from a first list of L1 ratio values and a second list of Alpha values.
Various embodiments are described, wherein training the linear regression model uses a genetic method wherein the set of hyperparameter pairs are randomly generated.
Various embodiments are described, wherein training the linear regression model further includes: sorting the R2 values associated with each pair of hyperparameters, wherein the set of hyperparameters includes N pairs of hyperparameters, wherein N is an integer; discarding I hyperparameter pairs in the set of hyperparameters with the lowest R2 values, where I is an integer less than N; randomly generating J hyperparameter pairs by randomly selecting L1 ratio values and Alpha values from other hyperparameter pairs in the set of hyperparameters, wherein J is an integer is less than I; randomly generating K hyperparameter pairs by randomly tweaking a randomly selected hyperparameter pair in the set of hyperparameters, wherein K is an integer less than I; and training the linear regression model using the set of updated hyperparameter pairs (L1, Alpha); and determining if the largest performance metric R2 has reached a global optimum.
Various embodiments are described, wherein the adherence index is a medication adherence index.
Various embodiments are described, wherein the demographic data includes one of age, income, insurance coverage, employment status, education level, housing status, and language status.
Various embodiments are described, wherein the clinical data includes one of medication duration, chronic condition; medication dosage, type of medication, allergies, and clinical outcome.
Various embodiments are described, further including: receiving data relating to an individual to be evaluated for adherence; and calculating an adherence index for the individual using the identified linear regression model based upon the received data relating to the individual.
Further various embodiments relate to a system for producing an adherence index model, including: a data extraction module configured to extract data for a group of individuals, wherein the extracted data includes demographic data and clinical data; and an adherence model generation module configured to train a linear regression model using a set of hyperparameter pairs (L1, Alpha), wherein the linear regression model produces an adherence index based upon the extracted data, the adherence model generation module further configured to: for each hyperparameter pair (L1, Alpha) in the set of hyperparameter pairs, train the linear regression model using a training data set to produce a linear regression model for each hyperparameter pair (L1, Alpha) and calculate a performance metric R2 for the resulting model based upon a validation data set, wherein the training data set is a subset of the extracted data and the validation data set is a subset of the extracted data that is different from the training data set; and identify the linear regression model with the largest performance metric R2.

Various embodiments are described, wherein the performance metric R2 is a measure the proportion of the variance in the adherence index that is predictable from the extracted data.

Various embodiments are described, wherein training the linear regression model uses a grid search wherein the set of hyperparameter pairs are generated from a first list of L1 ratio values and a second list of Alpha values.

Various embodiments are described, wherein training the linear regression model uses a genetic method wherein the set of hyperparameter pairs are randomly generated.

Various embodiments are described, wherein training the linear regression model further includes: sorting the R2 values associated with each pair of hyperparameters, wherein the set of hyperparameters includes N pairs of hyperparameters, wherein N is an integer; discarding I hyperparameter pairs in the set of hyperparameters with the lowest R2 values, where I is an integer less than N; randomly generating J hyperparameter pairs by randomly selecting L1 ratio values and Alpha values from other hyperparameter pairs in the set of hyperparameters, wherein J is an integer is less than I; randomly generating K hyperparameter pairs by randomly tweaking a randomly selected hyperparameter pair in the set of hyperparameters, wherein K is an integer less than I; and training the linear regression model using the set of updated hyperparameter pairs (L1, Alpha); and determining if the largest performance metric R2 has reached a global optimum.

Various embodiments are described, wherein the adherence index is a medication adherence index.

Various embodiments are described, wherein the demographic data includes one of age, income, insurance coverage, employment status, education level, housing status, and language status.

Various embodiments are described, wherein the clinical data includes one of medication duration, chronic condition; medication dosage, type of medication, allergies, and clinical outcome.

Various embodiments are described, further including an adherence index computation module that includes the identified linear regression model, configured to: receive data relating to an individual to be evaluated for adherence; and calculate an adherence index for the individual using the identified linear regression model based upon the received data relating to the individual.

## BRIEF DESCRIPTION OF THE DRAWINGS

In order to better understand various exemplary embodiments, reference is made to the accompanying drawings, wherein:

FIG. 1 illustrates a medication adherence system;
FIG. 2 illustrates the categories of data affecting medication adherence and hence the medication adherence index;
FIG. 3 illustrates a block diagram of the medication adherence model generation module;
IG. 4 illustrates a block diagram showing the operation of the medication adherence computation model; and
FIG. 5 illustrates a flow diagram of training the linear regression model.

To facilitate understanding, identical reference numerals have been used to designate elements having substantially the same or similar structure and/or substantially the same or similar function.

## DETAILED DESCRIPTION OF EMBODIMENTS

The description and drawings illustrate the principles of the invention. It will thus be appreciated that those skilled in the art will be able to devise various arrangements that, although not explicitly described or shown herein, embody the principles of the invention and are included within its scope. Furthermore, all examples recited herein are principally intended expressly to be for pedagogical purposes to aid the reader in understanding the principles of the invention and the concepts contributed by the inventor(s) to furthering the art and are to be construed as being without limitation to such specifically recited examples and conditions. Additionally, the term, "or," as used herein, refers to a non-exclusive or (*i.e.,* and/or), unless otherwise indicated (*e.g.,* "or else" or "or in the alternative"). Also, the various embodiments described herein are not necessarily mutually exclusive, as some embodiments can be combined with one or more other embodiments to form new embodiments.

The World Health Organization defines adherence as the extent to which a person's behavior, taking medications, following a diet, and/or executing lifestyle changes corresponds with agreed recommendations from a health care provider

The following are various types of medication non-adherence. Primary medication non-adherence includes when patients fail to pick-up or take newly prescribed medications. Secondary medication non-adherence occurs when the patient fills the prescription but does not take the prescription as prescribed (for example, delays in refills, cutting the dose, or reducing the frequency of taking the medication.) Non-adherence may also be intentional or unintentional. Intentional non-adherence is a rational decision to not take the medication, and unintentional non-adherence not taking medication due to forgetfulness or confusion. Medication persistence is another aspect of medication non-adherence where discontinuation of drug therapy occurs before the prescribed completion time.

Medication non-adherence has been a problem in the continuum of care as it adversely affects the patient's health and contributes to high healthcare costs. Identifying patients who are at high risk of non-adherence to medication is a challenge as it depends on various factors like clinical history, patient demographics, and other socio-economic factors.

There have been lot of studies in terms of factors affecting medication non-adherence. These studies have also come up with interventions that may have helped to improve adherence. In addition, there are solutions in the market that help patients to improve adherence by monitoring regular intake of medications. However, despite all these efforts, medication non-adherence continues to be a major problem that needs to be tackled in its early stages. Hence, there is a need for a screening tool to identify prospective non-adherent population. Such a population can be addressed with intervention systems that include medication adherence solutions (MAS).

MAS helps payers and healthcare delivery networks manage the costs of care for their high-acuity patients. In addition to reminding chronic disease patients to dispense their medications at pre-scheduled times, MAS provides daily notifications, monitoring, and reporting to pharmacists and organizations to help them manage patient medication adherence remotely.

Embodiments of a system and method for an artificial intelligence (AI) based approach for predicting a medication adherence index (MAI) for a subject based on clinical history, patient demographics, and socio-economic background will be described herein. The medication adherence index quantifies the risk for medication adherence. Lower values indicate higher risk of non-adherence. This system may be used as a pre-screening tool for providers to come up with better-tailored interventions for increasing medication adherence thereby improving the care quality and reduce healthcare costs.

The following are some more specific issues related to poor medication adherence. At any given time, ~50% of patients are non-adherent. Medication non-adherence in the U.S. costs $100 billion-$300 billion per year. 33%-69% of hospital admissions are due to non-adherence. Each of these are significant problems.

The embodiments of systems and methods described herein have the following advantages. The system and method may be used as a pre-screening tool for providers to come up with better-tailored interventions for increasing medication adherence thereby improving the care quality and reduce healthcare costs. The system and method may improve medication adherence for high-acuity, chronic disease patients, reduce unnecessary hospitalizations, and lower costs significantly for payers and providers.

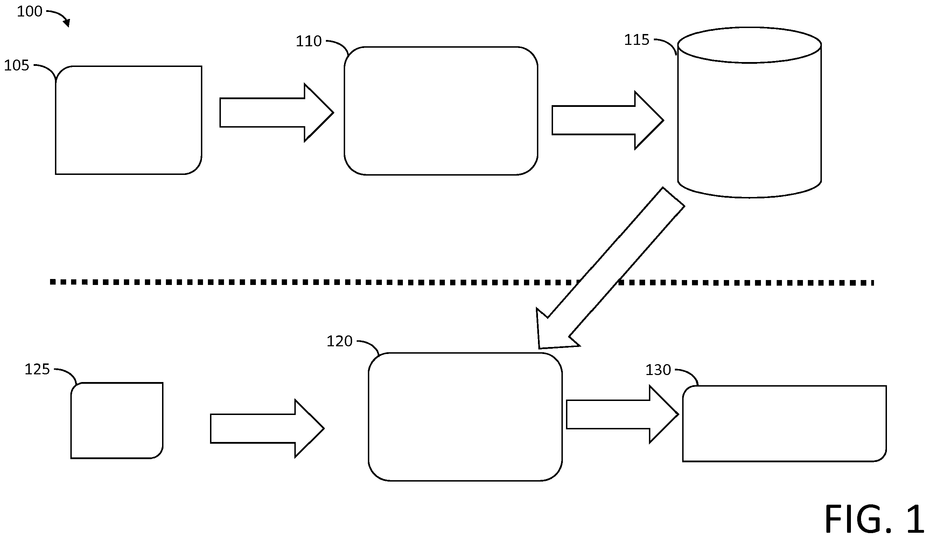

FIG. 1 illustrates a medication adherence system. The medical adherence system 100 has elements split into two phases. The first phase is the training phase in which a medication adherence index model 115 is generated. The second phase is the testing/deployment phase where the medication adherence index model 115 is implemented in a medication adherence index computation module 120 used to generate inferences.

The components of the medication adherence system 100 include the population electronic medical record (EMR) data 105, the medication adherence model generation module 110, the medication adherence index model 115, the medication adherence index computation module 120, the patient EMR data 125, and the patient medication adherence index 130. These will now be described in further detail. The medication adherence model generation module 110 receives population level EMR data 105 as training data for a machine-learning algorithm to generate a medication adherence index model 115. The medication adherence index computation module 120 takes as input patient level EMR data to compute a patient specific medication adherence index 130 using the medication adherence index model 115 for the computation.

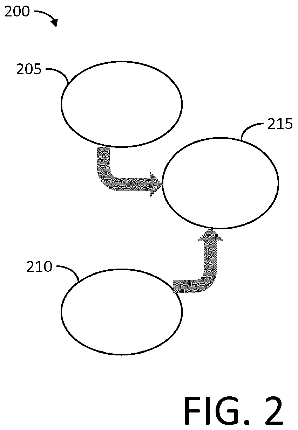

FIG. 2 illustrates the categories of data affecting medication adherence and hence the medication adherence index 215. The categories are patient demographics from the EMR database 205 and clinical data from the EMR database 210.

Regarding patient demographics from the EMR database 205, it is well established that the patient demographics/socio-economic factors affect the medication adherence. Various patient demographics that influence medication non-adherence will now be described.

Age (PDM1): old patients have cognitive difficulties and hence find it difficult to follow prescriptions and take medication on time.

Income (PDM2): low income patients are more prone to non-adherence as they might not be able to afford the cost of medications.

Insurance (PDM3): impacts medication affordability.

Employment Status (PDM4): impacts medication affordability.

Education (PDM5): patients with lower education levels have difficulty in comprehending medication instructions and the importance of adherence to medication regimen.

Housing (PDM6): patients living with family may have a higher medication adherence where those living alone may have a lower medication adherence.

Language (PDM7): patients who cannot speak the language in the country where they live may have difficulties in understanding the instructions regarding medication.

Features {PDM1, PDM2, PDM3, PDM4, PDM5, PDM6, and PDM7} constitute the patient demographic features. This list of demographic features is an example, and other demographic features having an influence on the medication adherence index may be used as well. Further, if another type of non-adherence is being modeled, then other demographic features that influence this other type of non-adherence may be used.

Patient clinical data from the EHR database 210 provides a holistic view of a patient's current chronic condition along with the medication prescribed. It may also provide in-depth medication details like medication type, duration, dosage *etc.* Various patient clinical data that influences medication non-adherence will now be described.

Medication duration (CD1): it has been observed that for longer the medication treatment duration, the probability of medication non-adherence increases.

Chronic conditions (CD2): the chronic condition that the patient is suffering from has an impact on medication non-adherence as some chronic conditions like hypertension have no symptoms when a dose or two is missed. The situation becomes more complex when co-morbidities are taken into consideration.

Medication Dosage/Complexity (CD3): multiple drugs with varied dosages will lead to confusions among patients. This may have an impact on medication non-adherence.

Type of medication (CD4): the medication delivery type may be a capsule, syrup, or injections. When multiple drugs are prescribed with different delivery types, this may lead to confusion among patients leading to non-adherence.

Allergy (CD5): patients who have minor adverse events to certain medications might not report back the adverse advents and tend to stop taking medicines when allergic reactions occur.

Clinical outcomes (CD6): clinical outcomes are tracked to find the effect of medication regiment on the patient health.

Pharmacy refills (TV): this data gives an idea about how much medication is ordered and whether it matches the prescription details. The measure may be a ratio of number of dosages consumed by patient over number of dosages prescribed by physician wherein the measure is in the range [0,1.0].

Features {CD1, CD2, CD3, CD4, CD5, CD6} constitute the clinical features and feature {TV} constitutes the target variable, where the desired value for the target variable is 1 or as close to 1 as possible.

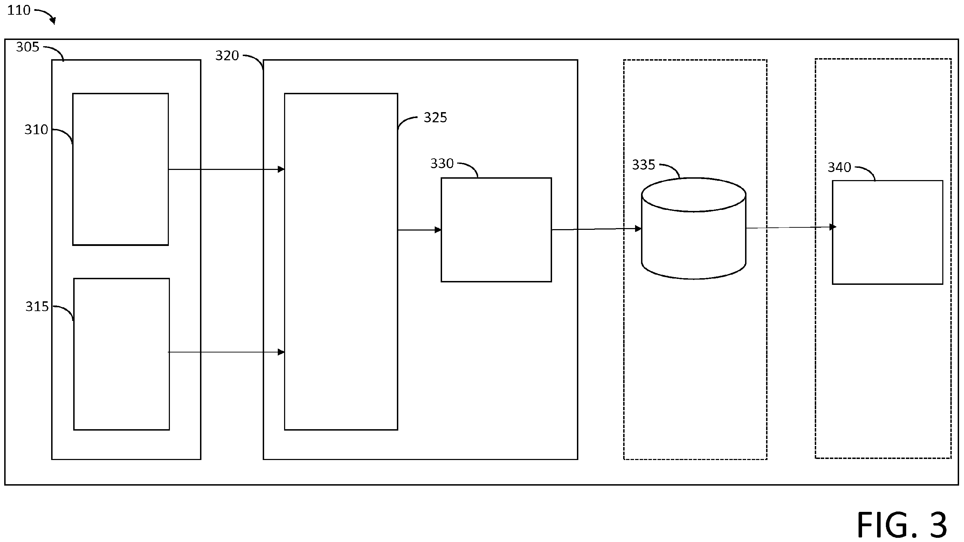

FIG. 3 illustrates a block diagram of the medication adherence model generation module 110. The medication adherence model generation module includes and EMR database 305, an extract-transform-load (ETL) component 320, a database 335, and a machine learning module 340.

The EMR database as described above may include the demographic data 310 and clinical data 315.

The ETL component 320 performs data preprocessing. The ETL component 320 includes a data extraction module 325 that extracts data from the EMR database 305. The ETL component 320 also includes a data normalization module 330 that normalizes the extracted data. The ETL component 320 then loads the transformed data to database 335. The machine-learning module 340 applies relevant techniques on this transformed data to build a predictive model.

In the data pre-processing step performed by the ETL component 320, historical data for all the relevant features are extracted from EMR database 305 for all available patient data. The pre-processing techniques applied to the features may include mean normalization and label encoding where the enumerated string values of features are converted to categorical INTEGER values. The example table below describes the features, data types and normalization method that may be applied to the features described above.

| Feature | Feature Label | Data Type | Normalization method |
|---|---|---|---|
| Age | PDM1 | INT | Mean Normalization |
| Income | PDM2 | FLOAT | Mean Normalization |
| Insurance | PDM3 | STRING(ENUMERATED) | Label Encoding |
| Employment status | PDM4 | STRING(ENUMERATED) | Label Encoding |
| Education | PDM5 | STRING(ENUMERATED) | Label Encoding |
| Housing | PDM6 | STRING(ENUMERATED) | Label Encoding |
| Language | PDM7 | STRING(ENUMERATED) | Label Encoding |
| Medication Duration | CD1 | INT | Mean Normalization |

(continued)

| Feature | Feature Label | Data Type | Normalization method |
|---------|---------------|-----------|----------------------|
| Chronic Condition | CD2 | STRING(ENUMERATED) | Label Encoding |
| Medication Dosage | CD3 | INT | Mean Normalization |
| Type of Medication | CD4 | STRING(ENUMERATED) | Label Encoding |
| Allergy | CD5 | STRING(ENUMERATED) | Label Encoding |
| Clinical Outcome | CD6 | STRING(ENUMERATED) | Label Encoding |
| Pharmacy Refills | TV | FLOAT | NOT APPLICABLE |

The machine learning module 340 takes as inputs the pre-processed features and trains a machine learning algorithm to generate a medication adherence index model 115. Data tuples of the following form may be created for each patient p in the range [1,n] where n is the total number of patients:

```
{PDM1(p[1]),PDM2(p[1]),..., PDM7(p[1]),CD1(p[1]),...,CD6(p[1]),TV(p[1])}.....
   {PDM1(p[n]),PDM2(p[n]),..., PDM7(p[n]),CD1(p[n]),...,CD6(p[n]),TV(p[n])}
```

This input is fed to a module which may implement an elastic net linear regression algorithm to train a linear regression model. In the process of training, the coefficient of determination is used to evaluate the accuracy of the predicted model. The coefficient of determination is denoted as $R2$ and is the proportion of the variance in the dependent variable that is predictable from the independent variable(s). Methods like grid-search or genetic algorithms may be used to determine the best values for the hyperparameters (L1 ratio, Alpha) of elastic net linear regression.

For instance, the grid search method initializes the values of L1 ratio = [0.9, 0.92, 0.95, 0.97, 0.99] and Alpha = [0.0125, 0.025, 0.05, .125, .25, .5, 1., 2., 4.]. Then 45 pairs of hyperparameters (L1 ratio, Alpha) are formed by combining each L1 ratio with each Alpha. This forms a "grid" of hyper parameters to use in training. $R2$ is used as the performance metric, which may be measured by cross-validation on the training set. In the grid search method, the various combination of the above hyperparameters are used for training the regression model, and the hyperparameter combination which generated highest $R2$ value is considered as the optimal model. The value $R2$ acts as a performance parameter for the models generated.

The genetic algorithm based hyperparameter optimization method is based on the evolution principle. In the genetic algorithm based hyperparameter optimization method, a population of genes may be defined. A fitness function may be defined for gene selection. Genes are allowed to mutate and cross populate for generating a new population. This process selects an optimal population using the following method:

1) Generate a random population of hyperparameter values. For example, a set of 100 value pairs. Each pair includes values for (Alpha, L1 ratio).
2) For each sample pair train the elastic net regression model using cross validation on the training set.
3) Compute the fitness function for each pair. The fitness function here is the $R2$ value.
4) Check if fitness value has reached global optimum; for example, if the difference in successive iterations of the $R2$ value is in the range of 0.0001. If the method has reached a global optimum, stop the process. Otherwise go on to step 5.
5) Sort the $R2$ values of the population in descending order. Select top 70 samples based upon $R2$ from the 100 samples. Out of these 70 sample randomly generate 20 new samples by mixing the parameter set by cross populating the parameters. For example, a new sample may be: sample[71]= (Alpha[25], L1ratio[1]). Next, randomly Generate another 10 samples from by tweaking the parameters by a small random positive/negative value. For example, sample [91] = (Alpha[1]+0.001, L1ratio[1]-0.001). These 100 sample constitute the new population. Proceed to Step 2 with the new population.

The optimal parameters are selected in step 4 when the method determines that a global optimum value for $R2$ has been found.

In linear regression, the model is represented as a collection of weights/coefficients of each feature along with an intercept.

The medication adherence index model is represented using the following values in the table below.

| Feature | Feature Label | Weights |
|---|---|---|
| Age | PDM1 | wPDM1 |
| Income | PDM2 | wPDM2 |
| Insurance | PDM3 | wPDM3 |
| Employment status | PDM4 | wPDM4 |
| Education | PDM5 | wPDM5 |
| Housing | PDM6 | wPDM6 |
| Language | PDM7 | wPDM7 |
| Medication Duration | CD1 | wCD1 |
| Chronic Condition | CD2 | wCD2 |
| Medication Dosage | CD3 | wCD3 |
| Type of Medication | CD4 | wCD4 |
| Allergy | CD5 | wCD5 |
| Clinical Outcome | CD6 | wCD6 |
| Intercept | INTC | A |

For a new patient "P" the medication adherence index may be computed as:

$$P[MAI] = \text{sum} (P[PDM1]*wPDM1 + P[PDM2]*wPDM2 + \ldots + P[PDM7]*wPDM7 +$$

$$P[CD1]*wCD1 + \ldots + P[CD6]*CD6 + A) *100.$$

This index is in the range of [0,100]. A lower value indicates high risk of non-adherence. A higher value indicates good medication adherence. The weights in the table above are parameters learned in the training of the machine learning model above.

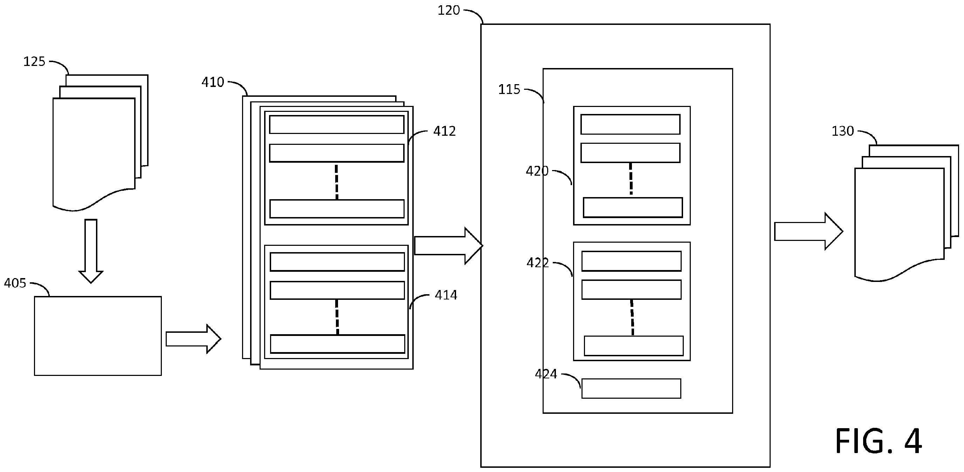

FIG. 4 illustrates a block diagram showing the operation of the medication adherence computation model 120. For new patients, EMR data for the patients is extracted and pre-processed by the pre-processing module 405. This results in the feature data such as the patient demographic features {PDM1, PDM2, PDM3, PDM4, PDM5, PDM6, and PDM7} 412 and the clinical features {CD1, CD2, CD3, CD4, CD5, CD6} 414. The input EMR data may be pre-processed by the data pre-processing module 405 as described in the previous section above.

For each patient "P" the medication adherence index 130 is computed by the medication adherence computation module 120 using the medication adherence index model 115 in the following manner:

$$P[MAI] = \text{sum}(P[PDM1]*wPDM1 + P[PDM2]*wPDM2 + \ldots + P[PDM7]*wPDM7 +$$

$$P[CD1]*wCD1 + \ldots + P[CD6]*CD6 + A) * 100$$

This index is in the range of [0,100]. A lower value indicates high risk of non-adherence. A higher value indicates good medication adherence.

This medical adherence system 100 may be used as a pre-screening tool for providers to come up with better-tailored interventions for increasing medication adherence, thereby improving the care quality and reduce healthcare costs.

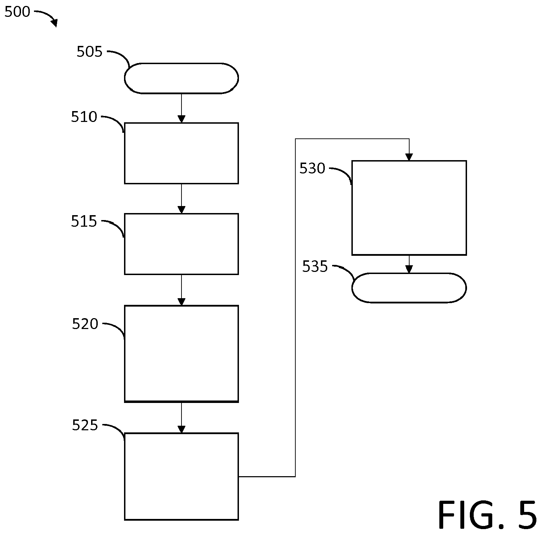

FIG. 5 illustrates a flow diagram of training the linear regression model. The training method 500 begins at 505 and then extracts data 510 as described above. Next, the training method 500 selects the hyperparameters to be used in training 515. This may be done using a grid search or genetic search as described above. Next, the training method trains the linear regression model using the different sets of hyperparameters 520. A performance metric R2 is next computed for each resulting model 525. Finally, the training method identifies the model with the largest performance metric 530, and then ends 535.

Further, while the specific examples given above were described using medication adherence, that medical adherence system may be applied to various types of prescribed treatment and lifestyle plans. In such cases, data features affecting the desired adherence may be used to train the machine learning model to generation an adherence index model that produces an adherence index.

The medical adherence system provides various technological benefits in identifying individuals that may be at risk for non-adherence to a treatment plan prescribed by a caregiver. The medical adherence system uses patient data to train a machine learning model to calculate an adherence index. This index indicates the risk that a patient may not adhere to a prescribed treatment plan. Hence, the medical adherence system now provides a solution to caregivers to identify patients as risk for non-adherence and provide additional resources to facilitate adherence.

The embodiments described herein may be implemented as software running on a processor with an associated memory and storage. The processor may be any hardware device capable of executing instructions stored in memory or storage or otherwise processing data. As such, the processor may include a microprocessor, field programmable gate array (FPGA), application-specific integrated circuit (ASIC), graphics processing units (GPU), specialized neural network processors, cloud computing systems, or other similar devices.

The memory may include various memories such as, for example L1, L2, or L3 cache or system memory. As such, the memory may include static random-access memory (SRAM), dynamic RAM (DRAM), flash memory, read only memory (ROM), or other similar memory devices.

The storage may include one or more machine-readable storage media such as read-only memory (ROM), random-access memory (RAM), magnetic disk storage media, optical storage media, flash-memory devices, or similar storage media. In various embodiments, the storage may store instructions for execution by the processor or data upon with the processor may operate. This software may implement the various embodiments described above.

Further such embodiments may be implemented on multiprocessor computer systems, distributed computer systems, and cloud computing systems. For example, the embodiments may be implemented as software on a server, a specific computer, on a cloud computing, or other computing platform.

Any combination of specific software running on a processor to implement the embodiments of the invention, constitute a specific dedicated machine.

As used herein, the term "non-transitory machine-readable storage medium" will be understood to exclude a transitory propagation signal but to include all forms of volatile and non-volatile memory.

Although the various exemplary embodiments have been described in detail with particular reference to certain exemplary aspects thereof, it should be understood that the invention is capable of other embodiments and its details are capable of modifications in various obvious respects. As is readily apparent to those skilled in the art, variations and modifications can be affected while remaining within the spirit and scope of the invention. Accordingly, the foregoing disclosure, description, and figures are for illustrative purposes only and do not in any way limit the invention, which is defined only by the claims.

**Claims**

1. A method for training an adherence model, the method comprising:

   extracting data for a group of individuals, wherein the extracted data includes demographic data and clinical data;
   training a linear regression model using a set of hyperparameter pairs (L1, Alpha), wherein the linear regression model produces an adherence index based upon the extracted data, further including:

   for each hyperparameter pair (L1, Alpha) in the set of hyperparameter pairs, training the linear regression model using a training data set to produce a linear regression model for each hyperparameter pair (L1, Alpha) and calculating a performance metric R2 for the resulting model based upon a validation data set, wherein the training data set is a subset of the extracted data and the validation data set is a subset of the extracted data that is different from the training data set; and

   identifying the linear regression model with the largest performance metric R2.

2. The method of claim 1, wherein the performance metric R2 is a measure the proportion of the variance in the adherence index that is predictable from the extracted data.

3. The method of claim 1, wherein training the linear regression model uses a grid search wherein the set of hyperparameter pairs are generated from a first list of L1 ratio values and a second list of Alpha values.

4. The method of claim 1, wherein training the linear regression model uses a genetic method wherein the set of hyperparameter pairs are randomly generated.

5. The method of claim 4, wherein training the linear regression model further includes:

sorting the R2 values associated with each pair of hyperparameters, wherein the set of hyperparameters includes N pairs of hyperparameters, wherein N is an integer;
discarding I hyperparameter pairs in the set of hyperparameters with the lowest R2 values, where I is an integer less than N;
randomly generating J hyperparameter pairs by randomly selecting L1 ratio values and Alpha values from other hyperparameter pairs in the set of hyperparameters, wherein J is an integer is less than I;
randomly generating K hyperparameter pairs by randomly tweaking a randomly selected hyperparameter pair in the set of hyperparameters, wherein K is an integer less than I; and
training the linear regression model using the set of updated hyperparameter pairs (L1, Alpha); and
determining if the largest performance metric R2 has reached a global optimum.

6. The method of claim 1, wherein the adherence index is a medication adherence index.

7. The method of claim 6, wherein the demographic data includes one of age, income, insurance coverage, employment status, education level, housing status, and language status.

8. The method of claim 6, wherein the clinical data includes one of medication duration, chronic condition; medication dosage, type of medication, allergies, and clinical outcome.

9. The method of claim 1, further comprising:

receiving data relating to an individual to be evaluated for adherence; and
calculating an adherence index for the individual using the identified linear regression model based upon the received data relating to the individual.

10. A system for producing an adherence index model, comprising:

a data extraction module configured to extract data for a group of individuals, wherein the extracted data includes demographic data and clinical data; and
an adherence model generation module configured to train a linear regression model using a set of hyperparameter pairs (L1, Alpha), wherein the linear regression model produces an adherence index based upon the extracted data, the adherence model generation module further configured to:

for each hyperparameter pair (L1, Alpha) in the set of hyperparameter pairs, train the linear regression model using a training data set to produce a linear regression model for each hyperparameter pair (L1, Alpha) and calculate a performance metric R2 for the resulting model based upon a validation data set, wherein the training data set is a subset of the extracted data and the validation data set is a subset of the extracted data that is different from the training data set; and

identify the linear regression model with the largest performance metric R2.

11. The system of claim 10, wherein the performance metric R2 is a measure the proportion of the variance in the adherence index that is predictable from the extracted data.

12. The system of claim 10, wherein training the linear regression model uses a grid search wherein the set of hyperparameter pairs are generated from a first list of L1 ratio values and a second list of Alpha values.

13. The system of claim 10, wherein training the linear regression model uses a genetic method wherein the set of hyperparameter pairs are randomly generated.

14. The system of claim 13, wherein training the linear regression model further includes:

sorting the R2 values associated with each pair of hyperparameters, wherein the set of hyperparameters includes N pairs of hyperparameters, wherein N is an integer;

discarding I hyperparameter pairs in the set of hyperparameters with the lowest R2 values, where I is an integer less than N;

randomly generating J hyperparameter pairs by randomly selecting L1 ratio values and Alpha values from other hyperparameter pairs in the set of hyperparameters, wherein J is an integer is less than I;

randomly generating K hyperparameter pairs by randomly tweaking a randomly selected hyperparameter pair in the set of hyperparameters, wherein K is an integer less than I; and

training the linear regression model using the set of updated hyperparameter pairs (L1, Alpha); and

determining if the largest performance metric R2 has reached a global optimum.

15. The system of claim 10, wherein the adherence index is a medication adherence index.

16. The system of claim 15, wherein the demographic data includes one of age, income, insurance coverage, employment status, education level, housing status, and language status.

17. system of claim 15, wherein the clinical data includes one of medication duration, chronic condition; medication dosage, type of medication, allergies, and clinical outcome.

18. The system of claim 10, further comprising an adherence index computation module that includes the identified linear regression model, configured to:

receive data relating to an individual to be evaluated for adherence; and

calculate an adherence index for the individual using the identified linear regression model based upon the received data relating to the individual.

FIG. 1

EP 3 654 340 A1

# FIG. 2

200

205

210

215

FIG. 3

FIG. 4

FIG. 5

**Europäisches Patentamt**
**European Patent Office**
**Office européen des brevets**

# EUROPEAN SEARCH REPORT

Application Number

EP 18 20 6265

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 9 773 094 B1 (BALAGERE DHANUR S [US] ET AL) 26 September 2017 (2017-09-26) * col. 1 lines 39-5 system, col. 12, lines 24-49 * ----- | 1-18 | INV. G16H20/10 |
| X | US 10 108 975 B1 (BENNER JOSHUA S [US] ET AL) 23 October 2018 (2018-10-23) * col. 5 line 26 - col. 6 line 9, col. 6 lines 40-65 * ----- | 1-18 | |

TECHNICAL FIELDS SEARCHED (IPC)

G16H

The present search report has been drawn up for all claims

| Place of search | Date of completion of the search | Examiner |
|---|---|---|
| Munich | 14 May 2019 | Bankwitz, Robert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 20 6265

This annex lists the patent family members relating to the patent documents cited in  the above-mentioned European search report.
The members are as contained in the European Patent Office EDP file on
The European Patent Office is in no way liable for these particulars which are merely given for the purpose of information.

14-05-2019

| Patent document cited in search report | | Publication date | Patent family member(s) | Publication date |
|---|---|---|---|---|
| US 9773094 | B1 | 26-09-2017 | NONE | |
| US 10108975 | B1 | 23-10-2018 | NONE | |

EPO FORM P0459

For more details about this annex : see Official Journal of the European Patent Office, No. 12/82